(19) Europäisches Patentamt
European Patent Office
Office européen des brevets

(11) Publication number: **0 333 020 A2**

# (12) EUROPEAN PATENT APPLICATION

(21) Application number: **89104144.4**

(22) Date of filing: **09.03.89**

(51) Int. Cl.4: **C07D 213/803**

(30) Priority: **18.03.88 US 169800**
**21.02.89 US 311871**

(43) Date of publication of application:
**20.09.89 Bulletin 89/38**

(84) Designated Contracting States:
**BE CH DE ES FR GB GR IT LI NL SE**

(71) Applicant: **ABBOTT LABORATORIES**
**One Abbott Park Road**
**Abbott Park, IL 60064-3500(US)**

(72) Inventor: **Cain, Michael H.**
**33256 N1 Mill Road**
**Wildwood Illinois 60030(US)**

(74) Representative: **Modiano, Guido et al**
**MODIANO, JOSIF, PISANTY & STAUB**
**Modiano & Associati Via Meravigli, 16**
**I-20123 Milan(IT)**

## (54) Process for the preparation of substituted pyridines.

(57) A process for the preparation of the substituted pyridines 2,6-dihydroxy-3-cyano-5-fluoropyridine, 2,6-dihydroxy-3-acetyl-5-fluoropyridine, 2,6-dichloro-3-cyano-5-fluoropyridine, 2,6-dichloro-5-fluoronicotinamide and 2,6-dichloro-5-fluoronicotinic acid.

EP 0 333 020 A2

# PROCESS FOR THE PREPARATION OF SUBSTITUTED PYRIDINES

## Technical Field

This is a continuation-in-part of U.S. patent application Serial No. 169,800, filed March 18, 1988.

This invention relates to a new process for the preparation of substituted pyridines useful in the production of pharmaceutical compositions, namely to the preparation of 2,6-dihydroxy-3-cyano-5-fluoropyridine, 2,6-dihydroxy-3-acetyl-5-fluoropyridine, 2,6-dichloro-3-cyano-5-fluoropyridine, 2,6-dichloro-5-fluoronicotinic acid and 2,6-dichloro-5-fluoronicotinamide.

## Background Art

Substituted pyridines are of particular interest as an intermediate for a number of organic syntheses, for example, in the synthesis of the naphthyridine antibacterial compounds described in U.S. Patent No. 4,616.019. According to the reaction scheme noted in the aforementioned patent, a nicotinic acid ester is hydrolyzed with a mineral acid to obtain the free acid, 2,6-dichloro-5-fluoronicotinic acid, as an intermediate. The nicotinic acid intermediate is further reacted to eventually give the disclosed naphthyridine antibacterial compounds.

Known processes for the preparation of substituted pyridines include the use of uracils as starting materials, for example, 1,3-dimethyluracil derivatives [J. Org. Chem., 46, 846-851 (1981)]; or the use of a 5-substituted-2,6-dichloro-3-methylpyridine [Helv. Chim. Acta, 59, 190 (1976),. and German Patent No. DE 3,508,816].

In one prior art scheme, the 1,3-dimethyluracil derivatives are reacted with malonamide to give a 5-substituted-2,6-dihydroxynicotinamide. See J Org. Chem., 46, 846-851 (1981).

In another scheme, the 5-amino-2,6-dichloro-3-methylpyridine is converted into 2,6-dichloro-5-fluoro-3-methylpyridine via 2,6-dichloro-3-methyl-5-(3,3-dimethyl-1-triazeno)pyridine. This product is then chlorinated to give 2,6-dichloro-5-fluoro-3-trichloromethylpyridine. Subsequent hydrolysis with sulfuric acid gives the carboxylic acid. See Helv. Chim. Acta, 59, 190 (1976); German Patent No. DE 3,508,816.

These prior art processes for the preparation of substituted pyridines require complex and expensive starting materials which are not always readily available in the quantities necessary for the manufacture of pharmaceutical products.

## Disclosure of the Invention

It has now been discovered that the substituted nicotinic acid 2,6-dichloro-5-fluoronicotinic acid may be prepared by the following reaction scheme.

$FCH_2C(O)OEt$ $HC(O)OEt$ $H_3CO^{\ominus}$ $Na^{\oplus}$

(a) ETHYL FLUOROACETATE + ETHYL FORMATE + SODIUM METHOXIDE

(1) (2) (3)

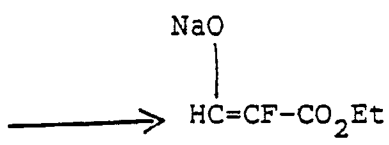

(4)

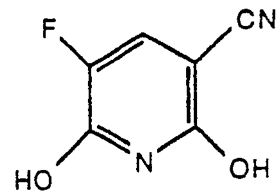

(b) 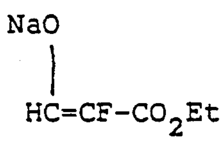

$NCCH_2C(O)NH_2$

+ CYANOACETAMIDE -> 2,6-DIHYDROXY-3-CYANO-5-FLUOROPYRIDINE

(4) (5) (I)

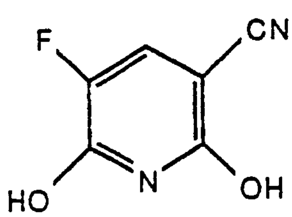

$POCl_3$ $PCl_5$

(c) 2,6-DIHYDROXY-3-CYANO-5-FLUOROPYRIDINE + PHOSPHORUS OXYCHLORIDE + PHOSPHORUS PENTACHLORIDE

(I) (6) (7)

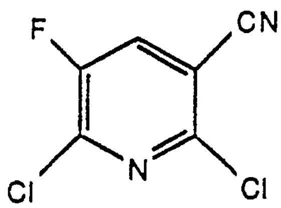

-> 2,6-DICHLORO-3-CYANO-5-FLUOROPYRIDINE

(II)

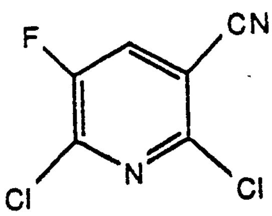

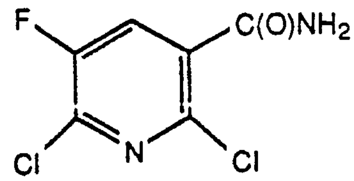

(d) 2,6-DICHLORO-3-CYANO-5-FLUOROPYRIDINE -> 2,6-DICHLORO-5-FLUORO-NICOTINAMIDE

(II) (8)

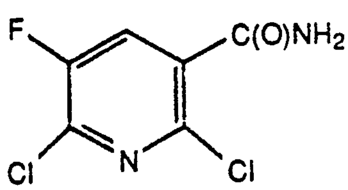

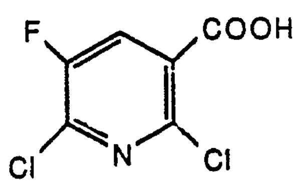

(e) 2,6-DICHLORO-5-FLUORO-NICOTINAMIDE -> 2,6-DICHLORO-5-FLUORONICOTINIC ACID

(8) (III)

2,6-Dihydroxy-5-fluoro-3-acetylpyridine and 2,6-dihydroxy-5-fluoronicontinamide may be prepared by the following scheme.

(f) 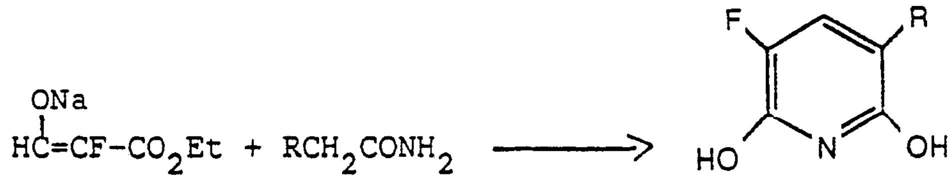

(4) R=$CONH_2$ (9) R=$COCH_3$ (10) R=$CONH_2$ (IV) R=$COCH_3$ (V)

In accordance with the foregoing reaction scheme, ethyl fluoroacetate (1) is reacted with ethyl formate (2) in the presence of a strong base such as sodium hydride, or in this case sodium methoxide (3), to form the enolate (4) of $OHCCHFCO_2Et$. The reaction can take place in an appropriate organic solvent which is preferably nonpolar, such as toluene. Since the condensation reaction is exothermic, the reagents are preferably added at a metered rate to prevent the heat of reaction from evaporating the ethyl formate (2). It is preferred that the temperature of the reaction mixture not exceed 30° C, since ethyl formate boils at 52-54° C. Alternatively, the heat of reaction can be removed by conventional techniques, such as by cooling the reaction vessel.

In step (b), cyanoacetamide (5) is added to the reaction mixture and the reaction allowed to proceed to form the product 2,6-dihydroxy-3-cyano-5-fluoropyridine (1). The mixture forms a thick suspension and, therefore, it is advantageous in proceeding with the reaction to add a suitable diluent, such as methanol. The product (I) is isolated from suspension by suitable methods, for example, by centrifugation.

In step (c), the product (I) is treated with an appropriate chlorinating agent such as $PCl_5$, $POCl_3$, $SOCl_2$ or $PCl_3$, preferably a mixture of $PCl_5$ and $POCl_3$, to displace the hydroxyl group and obtain the chlorinated product 2,6-dichloro-3-cyano-5-fluoropyridine (11). The product (II) is isolated by conventional solvent extraction techniques.

The cyano or nitrile group of the product (II) is hydrolyzed to form the carboxylic acid. This hydrolysis can be conducted in two steps, i.e., the nitrile is first hydrolyzed to the amide, which is then isolated and further hydrolyzed to the carboxylic acid. Alternatively, the nitrile can be directly hydrolyzed to the carboxylic acid in one step. The one step hydrolysis procedure is preferred.

The nitrile can be hydrolyzed directly to the carboxylic acid by treatment with a strong acid such as sulfuric acid, followed by treatment with hydrochloric acid. If the intermediate amide (8) is isolated, concentrated hydrochloric acid is then added and the amide is allowed to proceed to the carboxylic acid, 2,6-dichloro-5-fluoronicotinic acid (III). The crystalline product (III) is isolated, for example, by filtration or centrifugation.

As indicated in alternative reaction step f, other substituted acetamides [J. Org. Chem. 46 846-851 (1981)] will also function in this reaction to provide substituted pyridines. For example, (4) is treated with either malonamide (9) or acetylacetamide (10) to provide 2,6-dihydroxy-5-fluoronicotinamide (IV) or 2,6-dihydroxy-5-fluoro-3-acetylpyridine (V) respectively.

The foregoing description may be better understood from the following examples, which are presented for purposes of illustration and are not intended to limit the scope of the invention. In the following examples, all temperatures are set forth uncorrected in degrees Celsius and the references to compounds, such as (1), (2), (3), etc., refer to the corresponding compounds in the foregoing reaction scheme.

## Example 1

### 2,6-Dihydroxy-3-cyano-5-fluoropyridine (I)

In step (a), a 300 gallon reaction vessel was charged with 300 liter (L) of toluene, blanketed with nitrogen, and 30.6 kilograms (kg) of sodium methoxide (3) was added to the toluene in the reaction vessel Thereafter, 62.6 kg of ethyl formate (2) was added and the temperature was kept below 30° C. To this

mixture was added 30 kg of ethyl fluoroacetate (1). (Extreme caution should be observed when working with ethyl fluoroacetate. Ethyl fluoroacetate is highly toxic and supplied air gear should be worn to avoid breathing the vapors.) The ethyl formate and the ethyl fluoroacetate were added at metered rates to prevent the temperature of the reaction mixture from reaching a point where excessive evaporation of the reactants occurs.

Preferably, the temperature was not allowed to exceed approximately 30° C. This mixture was then stirred for 3 to 5 hours to allow the condensation reaction to proceed to the ester (4). At this point, the mixture was in the form of a suspension.

The suspension from step (a) was cooled to approximately 5-10° C and 71.4 kg of cyanoacetamide (5) was added. The resulting thick suspension was diluted with approximately 300 L of methanol, warmed to room temperature, and stirred approximately 12-16 hours to allow the reaction to proceed to completion. Glacial acetic acid (35 6 L) and water (200 L) were added to the suspension. The suspension was then centrifuged to separate the product (I) of step (b) from suspension, and the product (1) was dried. This product is the substituted pyridine 2,6-dihydroxy-3-cyano-5-fluoropyridine (I) having M.W. 154.10, m.p. 135-140° C. NMR (DMSO): 7.09 (1H, d, J = 12Hz).

## Example 2

## 2,6-Dichloro-5-fluoronicotinic acid (III)

In step (c), a 300 gallon reaction vessel was charged with 300 kg of phosphorus oxychloride (6), cooled, and blanketed with nitrogen. To the cooled phosphorous oxychloride (6), was added 182 kg of phosphorus pentachloride (7) and 30 kg of the dried 2,6-dihydroxy-3-cyano-5-fluoropyridine product (I) from step (b) The mixture was refluxed for 20 to 24 hours to allow the reaction to proceed to completion, forming the chlorinated product 2,6-dichloro-3-cyano-5-fluoropyridine (II) The mixture was cooled to room temperature and the phosphorus oxychloride was distilled off under vacuum Approximately 473 L of methylene chloride was added to the reaction vessel to dilute the suspension. The suspension containing the product (II) was then cooled to 5-10° C.

Elimination of phosphorous pentachloride was accomplished utilizing conventional extraction techniques. In this example, a second 300 gallon reaction vessel was charged with ice water (about 360 kg), the reactor jacket cooled to 0° C, and the methylene chloride suspension from the first vessel was slowly added to the second vessel thus decomposing the remaining phosphorous pentachloride. The mixture was then stirred and allowed to separate into layers. The methylene chloride layer, or lower organic layer which contains the product (II), was drained and transferred to the first reaction vessel The solution was dried and filtered before the methylene chloride was distilled off, leaving 2,6-dichloro-3-cyano-5-fluoropyridine (II) as an oily crystalline residue remaining in the reaction vessel. This product is the substituted pyridine 2,6-dichloro-3-cyano-5-fluoropyridine (II) having M.W. 190.99, m.p. 87-88° C. NMR (DMSO): 8.85 (1H, d, J = 8Hz) Mass spectrum (DEI): 190 (base peak).

The amide (8) was prepared in step (d) by adding concentrated sulfuric acid (185 kg) to the residue (II). The mixture was then heated to 75° C and held at this temperature for 1 hour. The sulfuric acid solution was then cooled and added slowly to 360 kg of ice water. The resulting suspension was extracted with 30% isopropyl alcohol/chloroform. The organic layer, containing the product (8) was then dried before the solvent was evaporated, leaving 2,6-dichloro-5-fluoronicotinamide (8). NMR (DMSO): 8.25 (1H, d, J = 8Hz), 8.13 (1H, s, broad), 7.98 (1H, s, broad).

Finally, hydrochloric acid (327 kg) was added to the product (8) and the mixture was refluxed for approximately 2 hours to allow the amide to hydrolyze to the carboxylic acid. The mixture was cooled and the crystalline product 2,8-dichloro-5-fluoronicotinic acid (III) was isolated by centrifugation and dried. This product is the substituted pyridine 2,6-dichloro-5-fluoronicotinic acid (III) havin, M.W. 209.99, m.p. 153-155° C NMR (DMSO): 8.41 (1H, d, J = 8Hz).

Alternatively, the following one-step hydrolysis of (II) can be accomplished. The nitrile (II) (28 g) was mixed with 26 ml of concentrated sulfuric acid. The mixture was heated to 75° C over a period of 45 minutes. The resulting solution was cooled on an ice bath and 130 ml of concentrated hydrochloric acid was added dropwise. This mixture was heated to reflux for 1 hour. The mixture was then allowed to cool to room temperature and was then cooled on an ice bath. The precipitate that formed was filtered to provide 6 gm of

the desired 2,6-dichloro-5-fluoronicotinic acid (III) as a tan crystalline solid.

## Example 3

### 2,6-Dihydroxy-3-cyano-5-fluoropyridine (Alternate preparation)

A one liter flask was charged with ethyl fluoroacetate (10 gm), ethyl formate (8.72 gm), ethanol (0.5 ml) and toluene (100 ml). The flask was cooled on an ice bath and sodium hydride (7.45 gm, 60% dispersion in mineral oil) was added in small portions. The mixture was stirred for thirty minutes. Cyanoacetamide (11.9 gm) in methanol (200 ml) was added over a 30 minute period to the sodium enolate. The solvent was evaporated under reduced pressure and water (100 ml) and acetic acid (12 ml) were added to the residue. The suspension was cooled on an ice bath and the precipitate was filtered to provide 2,6-dihydroxy-3-cyano-5-fluoropyridine (12.82 gm), m.p. 135-140° C NMR (DMSO): 7.09 (1H, d, J = 12Hz).

## Example 4

### 2,6-Dihydroxy-3-acetyl-5-fluoropyridine

A three neck one liter flask was charged with ethyl fluoroacetate (30 gm), ethyl formate (46 gm) and toluene (300ml). Sodium methoxide (30.6 gm) was added slowly. The temperature was maintained between 35° C and 45° C for five hours. Acetoacetamide in methanol (300 ml) was added to the thick suspension of the sodium enolate. This mixture was stirred for 24 hours at ambient temperature. The suspension was cooled on an ice bath and filtered. The solid obtained was dried to provide 41.4 gm of 2,6-dichloro-3-acetyl-5-fluoropyridine, m.p. 236-239° C. NMR (DMSO): 2.40 (3H, s), 7.72 (1H, d, J = 12Hz).

## Example 5

### 2,6-Dihydroxy-5-fluoronicotinamide

A 250 ml flask was charged with ethyl fluoroacetate (10 gm), ethyl formate (8.72 gm), ethanol 0.5 ml) and toluene (100 ml). Sodium hydride (7.45 gm, 60% dispersion in mineral oil) was added in small portions and the flask was cooled so that the temperature did nor go over 35° C. This mixture was stirred for 30 minutes and malonamide (14.4 gm) was added. Methanol (100 ml) was added and the mixture was stirred at ambient temperature overnight. Acetic acid 12 ml) was added to the suspension and the precipitate that formed was filtered, washed with water, followed by merhanol and then acetone to provide 2,6-dihydroxy-5-fluoronicotinamide (12.1 gm). This material was crystallized from acetic acid/methanol to provide crystalline amide, m.p. 300° C. NMR (DMSO): 7.83 (1H, d, J = 12Hz). Mass spectrum (CI, $NH_3$): 173 (M + H), 190 (M + $NH_4$).

The substituted pyridines prepared by the process of this invention are useful as intermediates for the preparation of antibacterial naphthyridine carboxylic acids. See Matsumoto, U.S. Patent No 4,649, 144; Chu, U.S Patent No. 4,616,019; and German Patent No. DE 3,508,816.

It will be understood that various changes and modifications can be made in the details of the procedure to adapt it to various conditions without departing from the spirit of the invention, especially as defined in the following claims.

## Claims

1. A process for the preparation of 2,6-dichloro-5-fluoronicotinic acid comprising:

(a) reacting ethyl fluoroacetate with ethyl formate in the presence of a base to form the enolate of $OHCCHFCO_2Et$;

(b) reacting the enolate formed in step (a) with cyanoacetamide to form 2,6-dihydroxy-3-cyano-5-fluoropyridine;

(c) chlorinating the 2,6-dihydroxy-3-cyano-5-fluoropyridine to form 2,6-dichloro-3-cyano-5-fluoropyridine; and

(d) hydrolyzing the 2,6-dichloro-3-cyano 5-fluoropyridine to form 2,6-dichloro-5-fluoronicotinic acid.

2. The process as defined in Claim 1 wherein the base is sodium methoxide or sodium hydride and the reaction temperature during step (a) is maintained at about 30 °C.

3. The process as defined in Claim 1 wherein the chlorinating agent is a combination of phosphorus oxychloride and phosphorus pentachloride.

4. The process as defined in Claim 1 wherein the 2,6-dichloro-3-cyano-5-fluoropyridine is hydrolyzed to directly obtain the 2,6-dichloro-5-fluoronicotinic acid.

5. The process as defined in Claim 1 wherein the 2,6-dichloro-3-cyano-5-fluoropyridine is hydrolyzed to obtain 2,6,-dichloro-5-fluoro-nicotinamide, which is isolated and thereafter hydrolyzed to obtain 2,6-dichloro-5-fluoronicotinic acid.

6. A process for the preparation of 2,6-dichloro-3-cyano-5-fluoropyridine comprising chlorinating 2,6-dihydroxy-3-cyano-5-fluoropyridine.

7. The process as defined in Claim 6 wherein the chlorinating agent is a combination of phosphorus oxychloride and phosphorus pentachloride.

8. A process for the preparation of 2,6-dichloro-5-fluoronicotinic acid comprising:

(a) chlorinating 2,6-dihydroxy-3-cyano-5-fluoropyridine to form 2,6-dichloro-3-cyano-5-fluoropyridine; and

(b) hydrolyzing the 2,6-dichloro-3-cyano-5-fluoropyridine to form 2,6-dichloro-5-fluoronicotinic acid.

9. The process of Claim 8 wherein the chlorinating agent is a combination of phosphorous oxychloride and phosphorous pentachloride.

10. The process of Claim 8 wherein the 2,6-dichloro-3-cyano-5-fluoropyridine is hydrolyzed to directly obtain the 2,6-dichloro-5-fluoronicotinic acid.